# EUROPEAN PATENT SPECIFICATION

(11) **EP 3 099 375 B1**
(45) Date of publication and mention of the grant of the patent: **22.04.2020**
(21) Application number: 14814849.7
(22) Date of filing: 17.12.2014
(51) Int. Cl.: A61M 39/22

(54) **OPENING DEVICE AND METHOD FOR OPENING A CLOSURE MEMBER AND ACTUATING DEVICES**
ÖFFNUNGSVORRICHTUNG UND VERFAHREN ZUM ÖFFNEN EINES VERSCHLUSSELEMENTS UND BETÄTIGUNGSVORRICHTUNGEN
PROCÉDÉ ET DISPOSITIF D'OUVERTURE POUR OUVRIR UN ÉLÉMENT DE FERMETURE ET DISPOSITIFS D'ACTIONNEMENT

(30) Priority: 31.01.2014 EP 14153462
(43) Date of publication of application: 07.12.2016
(73) Proprietor: Fresenius Kabi Deutschland GmbH, 61352 Bad Homburg (DE)
(72) Inventor: BRÜCKNER, Thomas, 63776 Mömbris (DE)
(74) Representative: Maikowski & Ninnemann Patentanwälte Partnerschaft mbB
(86) International application number: PCT/EP2014/078125
(87) International publication number: WO 2015/113701

(56) References cited:
- WO-A1-2012/177158
- US-A1- 2013 256 576

## Description

The invention relates to an opening device assembly for opening a closure member, in particular a closure member of a medical container according to claim 1, an actuating device for use with a drive mechanism of such an opening device assembly according to claims 7 and a method for opening a closure member according to claim 9.

Containers for liquids, for example medical containers (e.g. bags) for receiving a body substance (such as blood or blood components) often comprise a conduit or port that is blocked by a closure member, e.g. by a break-off valve that has to be broken to open the conduit or port. The closure member may be opened by manually applying a force to the closure member or by using an opening tool as disclosed in US 8,366,086 B2. Further, according to WO 2004/058046 A2 or US 2013/256576 A1, medical devices (such as blood preparation devices) using blood stored in blood bags may be equipped with an opening device (e.g. a breaker mechanism) for applying a force to a closure member to open a conduit or port of the blood bag initially blocked by the closure member. However, the opening device cannot be used with different types of blood bags.

It is an object of the invention to provide an opening device assembly that can be more flexibly used and to provide a corresponding actuating device and method for opening a closure member.

According to the invention, an opening device assembly comprising an opening device for opening a closure member, in particular a closure member of a medical container, and an actuating device for interacting with the closure member, is provided, the opening device comprising:
- a drive mechanism for driving the actuating device, wherein using the drive mechanism the actuating device can be moved from an initial position into an engagement position in order to open the closure member, and
- a connecting device for detachably connecting the actuating device to the drive mechanism such that the actuating device can be interchanged with another actuating device, wherein the connecting device is configured in such a way that at least a first and a second type of an actuating device can be operably connected to the drive mechanism, the first type using a rotational motion and the second type using a linear motion for interacting with the closure member, wherein
the actuating device comprises at least one actuating element configured for interacting with the closure member and an adapter element in the form of an adapter plate that is set into a linear motion by the drive mechanism, wherein the drive mechanism comprises at least one stud arranged eccentrically relative to a shaft of the drive mechanism, the stud interacting with the adapter element when the actuating device is connected to the drive mechanism, and wherein the actuating element is coupled to the adapter element in such a way that the linear motion of the adapter element is transferred to the actuating element or causes a rotational motion of the actuating element, and wherein the actuating device comprises a rotatable member having two actuating elements in the form of arms and an eccentric pin that engages a longitudinal recess of the adapter element such that the to and fro movement of the adapter element causes a back and forth rotation of the rotatable member and thus of the arms.

The connecting device in particular permits the actuating device to be attached to the drive mechanism and removed from the drive mechanism manually, i.e. without having to use a tool. Thus, the opening device according to the invention allows the actuating device to be easily interchanged with another actuating device such that the opening device can be used to open different types of closure members and/or containers comprising the closure member.

For example, the actuating device is adapted to a certain position of the closure member relative to the container's periphery, wherein it might be replaced by another actuating device that is adapted to another position of the closure member. Also, the actuating device whose actuating element moves linearly (e.g. in order to squeeze the closure member) may be replaced by an actuating device whose actuating element will be rotated by the drive mechanism in order to open closure members that require the application of a shear force for being opened.

For example, the connecting device of the opening device comprises at least one longitudinal element fixedly connected to the drive mechanism and a corresponding opening of the actuating device into which the longitudinal element is inserted when connecting the actuating device to the drive mechanism. For example, the opening of the actuating device mates with the longitudinal element such that a (detachable) tight fit between the longitudinal element and the opening may be realized. In addition or as an alternative, other connecting elements may be provided, e.g. for realizing a latching connection between the drive mechanism and the actuating device.

According to another embodiment of the invention, the drive mechanism comprises at least one stud arranged eccentrically relative to a shaft of the drive mechanism, the stud interacting with the actuating element when the actuating device is connected to the drive mechanism. In particular, the stud conveys the movement generated by the drive mechanism to the actuating element of the actuating device. The conversion of the rotational movement provided by the drive mechanism into a linear movement may be realized by arranging the actuating element in a guiding element as will be explained in more detail below.

For example, the drive mechanism comprises two studs arranged eccentrically relative to a shaft of the drive mechanism, wherein one of the studs is shorter (measured in the shaft direction) than the other one. The different length of the studs permits the usage of a plurality of actuating devices, wherein some of the actuating devices make use of both of the studs to generate a movement of their actuating element and others only use one of the studs (the longer one), wherein the shorter stud will also set into motion by the drive mechanism but will not be operationally coupled to the actuating device.

The invention also relates to a medical device, in particular a blood preparation device, comprising an opening device as described above for automatically opening the closure member of a blood bag.

The actuating device may comprise at least one actuating element configured for interacting with the closure member and at least one connecting element for being detachably connected to the drive mechanism of the opening device, and wherein the actuating device is configured in such a way that a rotational movement provided by the drive mechanism is converted into a linear movement of the actuating element.

The actuating device may comprise at least one actuating element configured for interacting with the closure member and at least one connecting element for being detachably connected to the drive mechanism of the opening device, and wherein the actuating device is configured in such a way that a rotational movement provided by the drive mechanism causes a rotational movement of the actuating element.

According to another aspect of the invention, the actuating device comprises two actuating elements in the form of two arms that can be rotated into the engagement position. The second actuating device may further comprise two fixed protrusions arranged in such a way that the closure member will be bent when the two arms are rotated into the engagement position, wherein the actuating device comprises a first pair of actuating elements that is set in a rotational motion by a rotational movement provided by the drive mechanism and a second pair of actuating elements that is set in a linear motion by a rotational movement provided by the drive mechanism. In particular, the first and the second pair of actuating elements are moved in opposite directions.

For creating the movement of the actuating element(s) the actuating device may comprise an adapter element (e.g. an adapter plate) that is set in a linear motion by the drive mechanism of the opening device, wherein the actuating element is coupled to the adapter element in such a way that the linear motion of the adapter element causes a rotational motion of the actuating element or is transferred to the actuating element (in that case the linear motion of the adapter element causes a linear motion of the actuating element). For example, the adapter element is configured in such a way that the actuating element can be arranged at different positions relative to the adapter element.

The opening device assembly may comprise
- an opening device as described above for opening a closure member and at least a first and a second actuating device that can be interchangeably connected to the drive mechanism of the opening device via the connecting device,
- wherein the first actuating device is an actuating device that comprises at least one actuating element configured for interacting with the closure member and at least one connecting element for being detachably connected to the drive mechanism of the opening device, and wherein the actuating device is configured in such a way that a rotational movement provided by the drive mechanism is converted into a linear movement of the actuating element.

The second actuating device may be an actuating device that comprises at least one actuating element configured for interacting with the closure member and at least one connecting element for being detachably connected to the drive mechanism of the opening device, wherein the actuating device is configured in such a way that a rotational movement provided by the drive mechanism causes a rotational movement of the actuating element.

Thus, depending on the configuration of the closure member (or the container comprising the closure member) one of the actuating devices of the opening device assembly is chosen and connected to the drive mechanism. Due to the detachable connection, the actuating device can be replaced by another actuating device of the opening device assembly adapted to another closure member having a different configuration (e.g. size, position and/or type).

The invention is also related to a method for opening a closure member, in particular a closure member of a medical container, comprising the steps of:
- providing an opening device assembly as described above;
- choosing one of the actuating devices of the opening device assembly dependent on the configuration of the closure member; and
- connecting the chosen actuating device to the drive mechanism.

For example, an actuating device is removed from the drive mechanism and replaced by another actuating device of the opening device assembly.

Embodiments of the invention are discussed hereinafter with reference to the drawings, in which:
- Figure 1A: shows a portion of blood preparation device comprising an opening device according to an embodiment of the invention;
- Figure 1B: shows an actuating device for use with the opening device shown in figure 1A according to an embodiment of the invention;
- Figure 1C: depicts an actuating device according to another embodiment of the invention for use with the opening device shown in figure 1A;
- Figures 2A - 2D: illustrate embodiments of an actuating device according to the invention;
- Figure 3: shows a sectional view of a drive mechanism of an opening device according to the invention;
- Figure 4: illustrates the drive mechanism of Figure 3, wherein an actuating device is arranged at the drive mechanism;
- Figures 5A - 5I: schematically illustrate configurations of an actuating device according to the invention; and
- Figure 6: schematically depicts an actuating device according to the invention in the engagement position.

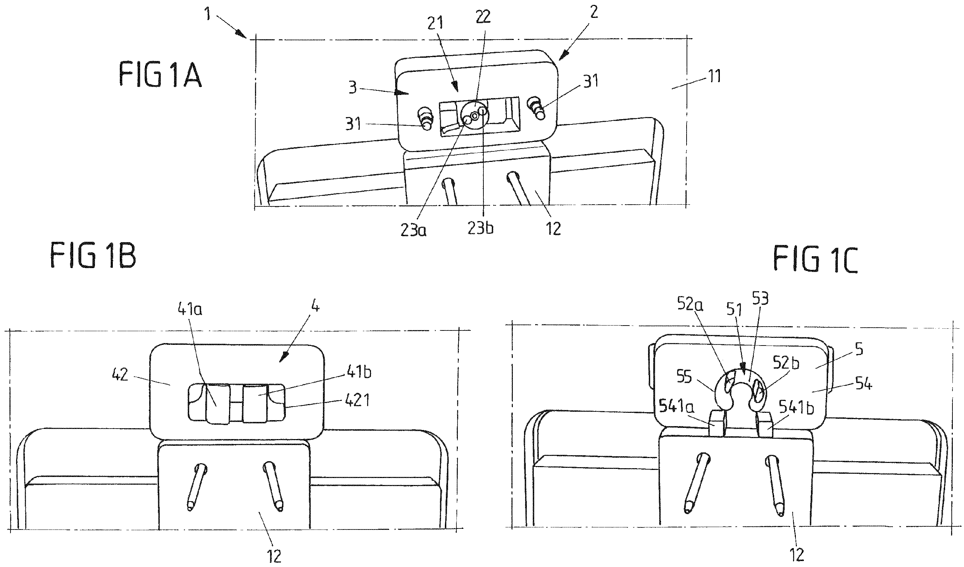

Figure 1A shows a perspective view of a portion of a medical device in the form of a blood preparation device 1. The blood preparation device 1 comprises a housing 11 and an attachment region 12 for receiving a blood container (blood bag). Above the attachment region 12 a drive mechanism 21 of an opening device 2 for opening a closure member of the blood bag (not shown) is arranged, the closure member blocking a conduit (e.g. a tube) or port of the blood bag for extracting blood from the blood bag. The drive mechanism 21 comprises a driver unit such as an electro-motor for driving a shaft. The driver unit and the shaft are arranged within the housing 11 and thus are not visible in Fig. 1A. The shaft of the drive mechanism 21 is connected to a rotatable disc 22 having two eccentrically arranged studs 23a, 23b.

The drive mechanism 21 is used to drive an actuating device (not shown in Fig. 1A) configured for interacting with the closure member of the blood bag. In order to be able to use different types of blood bags (e.g. from different manufacturers), which possess different types (e.g. with respect to the size and/or position) of the closure member, the opening device 2 comprises a connecting device in the form of a universal mount 3. The universal mount 3 is configured to allow different types of actuating devices (e.g. adapted to different blood bag types) to be connected to drive mechanism 21 such as the actuating devices 4 and 5 shown in Figures 1B and 1C.

For this, the universal mount 3 comprises two longitudinal elements in the form of bolts 31 engaging into corresponding openings of the actuating devices. Thus, the actuating devices can be detachably connected to the drive mechanism 21 via the universal mount 3. Further, the universal mount 3 transfers a rotation generated by the drive mechanism 21 to the actuating device in such a way that both an actuating device using a rotational motion and an actuating device using a linear motion for interacting with the closure member can be operated by means of the drive mechanism 21.

The actuating device 4 shown in Fig. 1B comprises two actuating elements 41a, 41b (e.g. in the form of jaws), wherein the actuating device 4 is configured in such a way that a rotational movement of the shaft of the drive mechanism 21 is converted into a linear movement of the actuating elements 41a, 41b. For this, the actuating device 4 comprises a guiding element 42 having a rectangular opening 421 linearly guiding the actuating elements 41a, 41b. Using the guiding opening, the actuating elements 41a, 41b are linearly moved from an initial position towards one another into an engagement position when the drive mechanism 21 is activated. It is also possible that only one of the actuating elements 41a, 41b moves towards the other one (which in that case does not move).

For opening the closure member of the blood bag, the blood bag is arranged at the attachment region 12 in such a way that a region with the closure member is arranged between the actuating elements 41a, 41b of the actuating device 4. Thus, by means of the driving mechanism 21 at least one of the actuating elements 41a, 41b is moved towards the closure member and presses the closure member towards the other actuating element such that the closure member breaks or is deformed in such a way that it does not block the conduit or passage of the blood bag anymore.

As already set forth above, instead of the actuating device 4 depicted in Fig. 1B, another actuating device can be used for opening a closure member of a different blood bag type. For example, an actuating device 5 shown in Fig. 1B could be used, wherein similar to actuating device 4 shown in Fig. 1B, actuating device 5 comprises openings in its back side such that it can be detachably connected to the bolts 31 of the universal mount 3 of opening device 2. However, unlike the actuating device 4 of Figure 1B, actuating device 5 does not convert the rotational movement provided by the drive mechanism 21 into a linear movement.

Rather, actuating device 5 comprises a rotatable member 51 that can be rotated by means of the drive mechanism 21, the rotatable member 51 comprising two actuating elements in the form of arms 52a, 52b protruding from a base body 53 of the rotatable member 51.

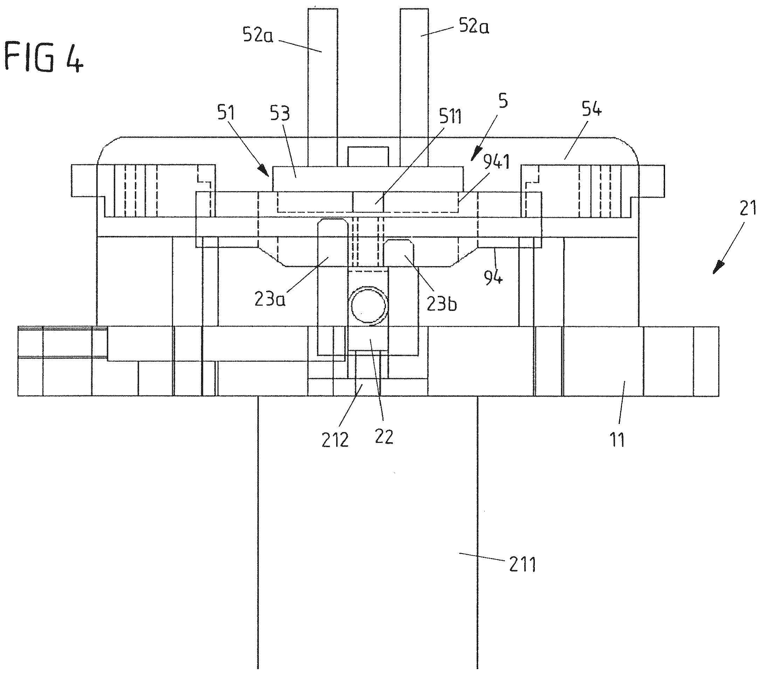

Further, the actuating device 5 comprises an adapter element in the form of an adapter plate (not visible in Fig. 1C) arranged between the rotatable member 51 and the drive mechanism 21 (see Fig. 4 adapter plate 94). More particularly, the adapter plate interacts with the studs 23a, 23b of the drive mechanism 21 in such a way that it is set into a linear to and fro movement by the rotational movement generated by the drive mechanism 21. The adapter plate, in turn, in a side facing towards the rotatable member 51 comprises a longitudinal (vertical) recess receiving an eccentric pin (not visible in Fig. 1B, either) such that the linear movement of the adapter plate is converted into a rotational movement of the rotatable member 51 and thus of the arms 52a, 52b.

The actuating device 5 moreover comprises a housing 54, wherein fixed protrusions 541a, 541b are arranged on an outer surface of the housing 54 and the arms 52a, 52b reach through an opening 55 of the housing 54. The blood bag is arranged in such a way that a region containing the closure member will be arranged between the fixed protrusions 541a, 541b and the rotatable arms 52a, 52b. Thus, by rotating the rotatable member 51 (by means of the drive mechanism 21), an upper part of the closure member region will be rotated while a lower part of the closure member region will be held in place by the protrusions 541a, 541b. Hence, by activating the drive mechanism 21 a relative movement between the upper and the lower part of the closure member region is created, which breaks or deforms the closure member and thus unblocks the passage or port of the blood bag initially blocked by the closure member.

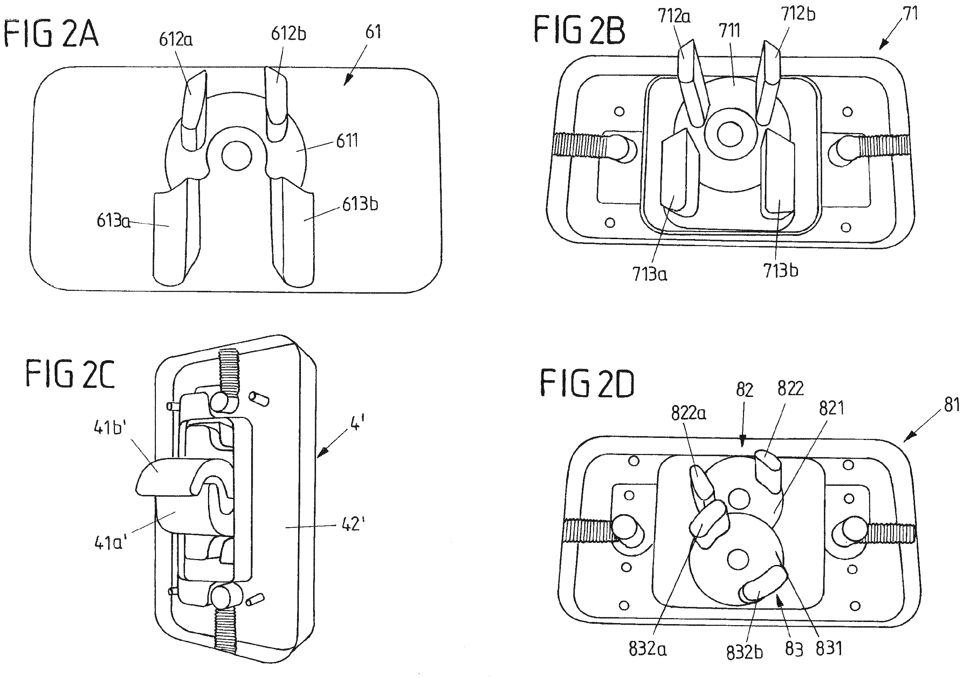

Figures 2A to 2D show further examples of actuating devices that can be connected to the drive mechanism 21 of the opening device 2 by means of the universal mount 3. The actuating device 61 shown in Figure 2A resembles the actuating device 5 shown in Figure 1C as it comprises a rotatable base body 611 having arms 612a, 612b and fixed protrusions 613a, 613b.

Similarly, the actuating device 71 shown in Figure 2B comprises arms 712a, 712b connected to a rotatable base body 711 and fixed protrusions 713a, 713b below the arms 712a, 712b. Figure 2C shows another example of an actuating device 4' similar to the one shown in Figure 1B, i.e. the actuating device 4' comprises arms 41a', 41b' linearly guided in a guiding element 42'.

The actuating device 81 shown in Figure 2D comprises two rotatable members 82, 83, each of them having a (e.g. ring or circular shaped) base body 821, 831. Arms 822a, 822b and 832a, 832b protrude from the base body 821, 831, respectively.. The upper rotatable member 82 will be set in rotation by the drive mechanism of the opening device as explained above with respect to the rotatable member 51 shown in Fig. 1C. Further, the upper rotatable member 82 interacts with the lower rotatable member 83 such that the rotational motion of the upper rotatable member 82 induces a counter rotation of the lower rotatable member 83. For example, a protrusion of the upper rotatable member 82 engages an opening of the lower rotatable member 83. In an alternative configuration, the lower rotatable member 83 is directly driven by the drive mechanism and generates the counter rotation of the upper rotatable member 82. Due to the opposite rotation of the rotatable members 82, 83 the closure member region of a blood bag is bent until the closure member breaks or is deformed in such a way that blood can flow through the conduit formerly blocked by the closure member.

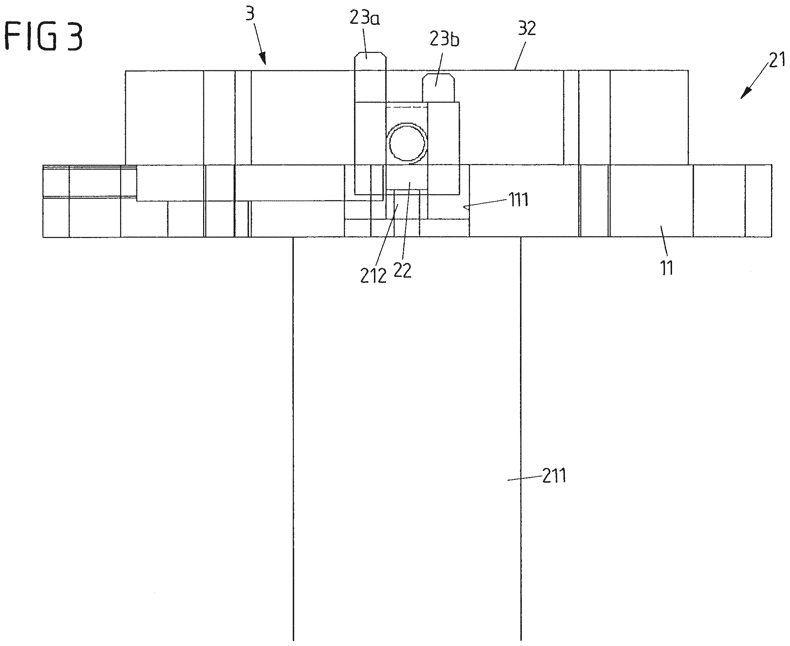

Figure 3 shows a schematic sectional view of the driving mechanism 21 and the universal mount 3 of the opening device shown in Fig. 1A. The driving mechanism 21 comprises a driving motor 211 driving a shaft 212 for generating a rotational movement of the rotatable disc 22. As mentioned above, the rotatable disc 22 carries two studs 23a, 23b for conveying the rotational motion provided by the drive mechanism 21 to the actuation device (not shown in Fig. 3).

The motor 211 is arranged within a portion of the housing 11 of the blood preparation device, wherein the shaft 212 and the rotating plate 22 reach through an opening 111 of the housing 11 such that the studs 23a, 23b are accessible from the outside of the housing 11. A section of the housing 11 forms a bearing surface 32 of the universal mount 3, wherein an actuating device may be arranged at the universal mount 3 in such a way that a backside (facing away from the actuating element) gets in contact with the bearing surface 32.

An example of how an actuating device 5 is connected to the universal mount 3 is shown in the schematic sectional view of Figure 4. In this case, an actuating device 5 similar to the one shown in Figure 1C is detachably arranged at the universal mount 3 and thus at the driving mechanism 21. The actuating device 5 comprises a rotatable member 51 having gripper arms 52a, 52b as actuating elements protruding from a base body 53.

The studs 23a, 23b of the driving mechanism 21 interact with an adapter plate 94 in such a way that due to a rotation of the studs 23a, 23b the adapter plate 94 will carry out a to and fro movement. The adapter plate 94 in a side facing towards the rotatable member 51 further comprises a longitudinal recess 941 into which an eccentric pin 511 of the rotatable member 51 engages such that the to and fro movement of the adapter plate 94 causes a back and forth rotation of the rotatable member 51 and thus of the arms 52a, 52b.

It is also possible that an opening device assembly is provided, the assembly comprising an opening device 2 (or a medical device comprising such an opening device) and different actuating devices for use with the drive mechanism 21 of the opening device 2. For example, the opening device assembly comprises different kinds of actuating devices (such as those shown in Figure 2A-2D).

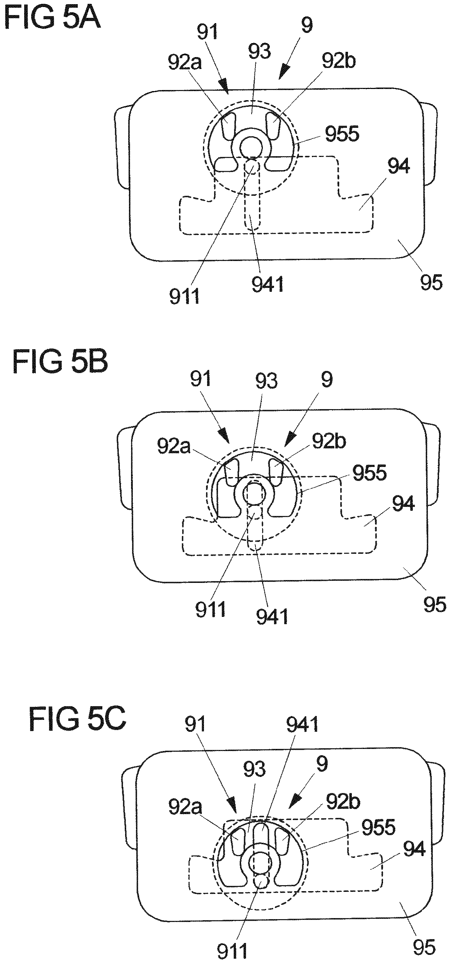

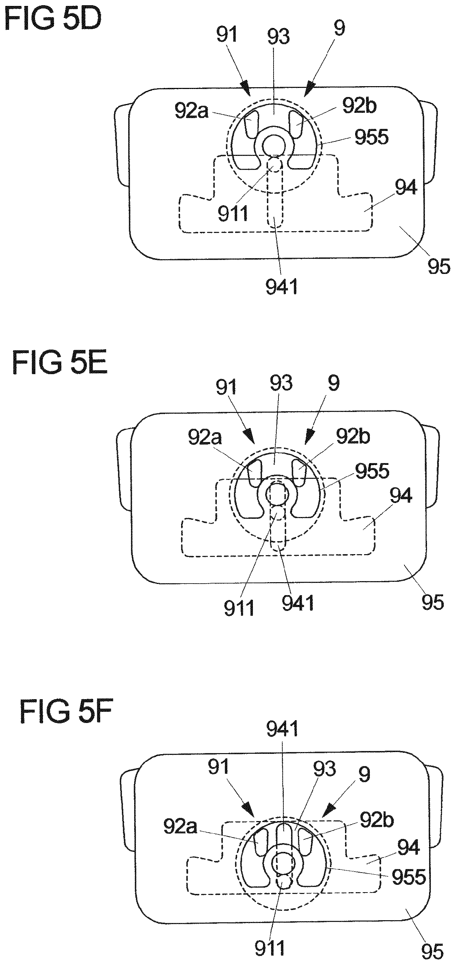

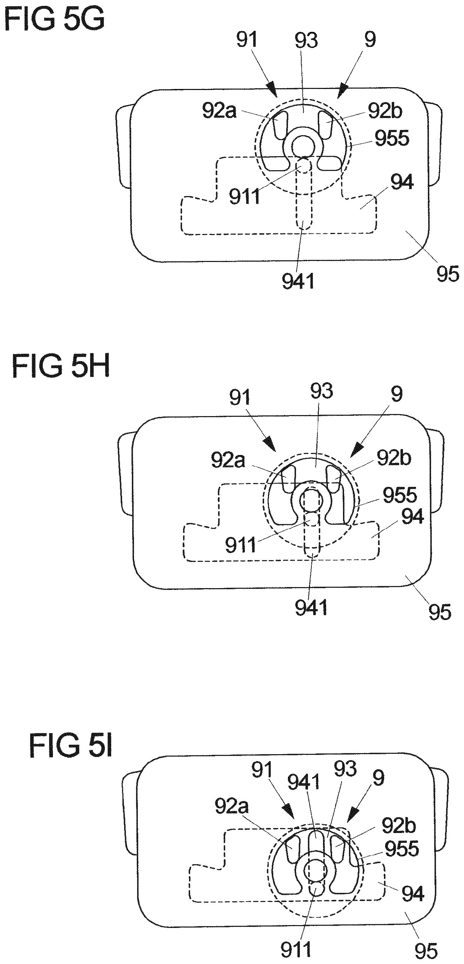

Fig. 5A to 5I illustrate an actuating device 9 configured similarly to the actuating device 5 of Fig. 1C, i.e. the actuating device 9 comprises a rotatable member 91 and two arms 92a, 92b connected to a rotatable base body 93.

Further, the actuating device 9 comprises an adapter plate 94, the rotatable member 91 and the adapter plate 94 being arranged in a housing 95, wherein the arms 92a, 92b reach through an arcuated opening 955 of the housing 95. The adapter plate 94 as explained above with respect to Fig. 1C and 4 interacts with a pin 911 (engaging a recess or notch 941 of the adapter plate 94) such that a linear back and forth movement (produced by the drive mechanism of the opening device) generates a rotational back and forth movement of the rotatable member 91. The recess 941 has a longitudinal shape extending in the vertical direction (perpendicular to the movement of the plate 94) such that the rotatable member 91 can be arranged at different (height) positions relative to the adapter plate 94 while the pin 911 still engages the recess 941. Thus, the vertical position of the rotatable member 91 and thus the vertical position of the gripper arms 92a, 92b relative to the drive mechanism of the opening device can be adapted to different blood bag types, wherein for each vertical position of the rotatable member 91 different housings 95 (having different vertical positions of the arcuated opening 955) are used as shown in Fig. 5A - 5C, 5D - 5F and 5G - 5I, respectively. However, it is also conceivable that the same housing is used, wherein for adapting the vertical position of the arms 92a, 92b the rotatable member 91 is vertically displaced together with the housing 95 relative to the adapter plate 94.

Moreover, different adapter plates 94 could be provided, wherein the horizontal position of the recess 941 varies. For example, the recess 941 may be located in the center (Fig. 5D-F) of the adapter plate 94, with an offset to the left relative to the (horizontal) center of the adapter plate 94 (Figures 5A-5C) or to the right of the center of the adapter plate 94 (Figures 5G-5I). For example, an opening device assembly comprising an opening device according to the invention and different adapter plates (such as the adapter plates 94 shown in Fig. 5A-5C, 5D-5F or 5G-5I) might be provided.

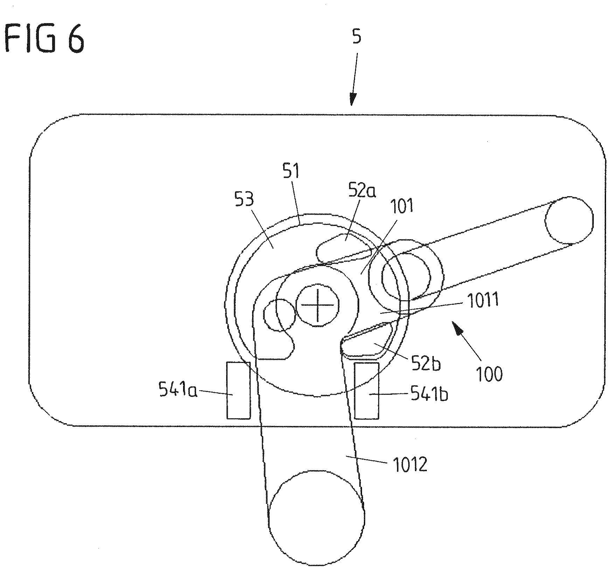

Figure 6 schematically shows the actuating device 5 of Figure 1C, wherein a region of a conduit 100 (e.g. of a blood bag) comprising a closure member 101 is arranged between the arms 52a, 52b of the rotatable member 51 of the actuating device 5. The rotatable member 51 and thus the arms 52a, 52b were rotated from an initial position into an engagement position, i.e. the arms 52a, 52b were rotated relative to the fixed protrusions 541a, 541b such that an upper part 1011 of the closure member 101 will be bent relative to a lower part 1012, thereby deforming or breaking the closure member 101, thereby unblocking the conduit 100.

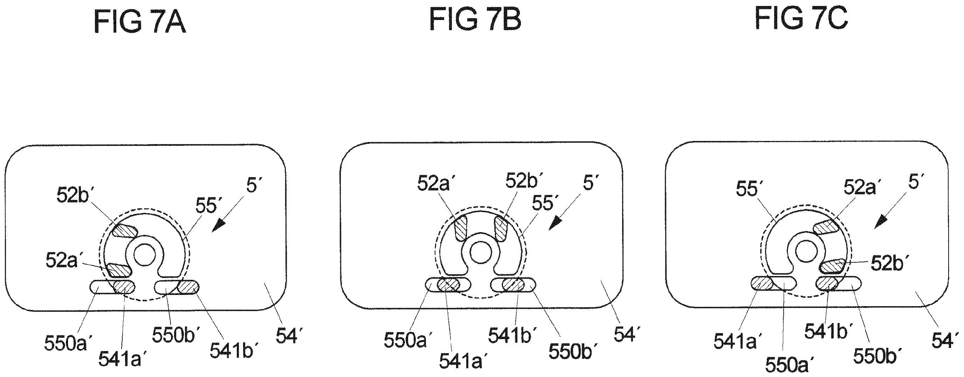

Fig. 7A to 7C illustrate another embodiment of an actuating device 5' according to the invention. The actuating device 5' similar to the embodiment shown in Fig. 1C and Fig. 4 comprises actuating elements in the form of a two gripper arms 52a', 52b' formed by a rotatable member 51'. A rotation of the rotatable member 51' is generated by means of an adapter plate (not visible in Fig. 7A to 7C). The arms 52a', 52b' reach through an arcuated opening 55' of a housing 54'.

However, instead of additional fixed protrusions, the actuating device 5' comprises two further movable arms 541a', 541b' which are set in a linear motion by a movement of the adapter plate (i.e. by a rotation generated by the driving mechanism 21). More particularly, the movable arms 541a', 541b' are moved in an opposite direction with respect to the rotation of the arms 52a', 52b', i.e. when the gripper arms 52a', 52b' rotate counter-clockwise, the movable arms 541a', 541b' move to the right, (Fig. 7A) with respect to an initial position (shown in Fig. 7B). Conversely, when the gripper arms 52a', 52b' rotate clockwise, the movable arms 541a', 541b' are moved to the left (Fig. 7C). The movable arms 541a', 541b' each reach through further openings 550a', 550b' of the housing 54'. The additional movement of the additional movable arms 541a', 541b' may increase the strain of the closure element and thus may accelerate the opening of the closure element.

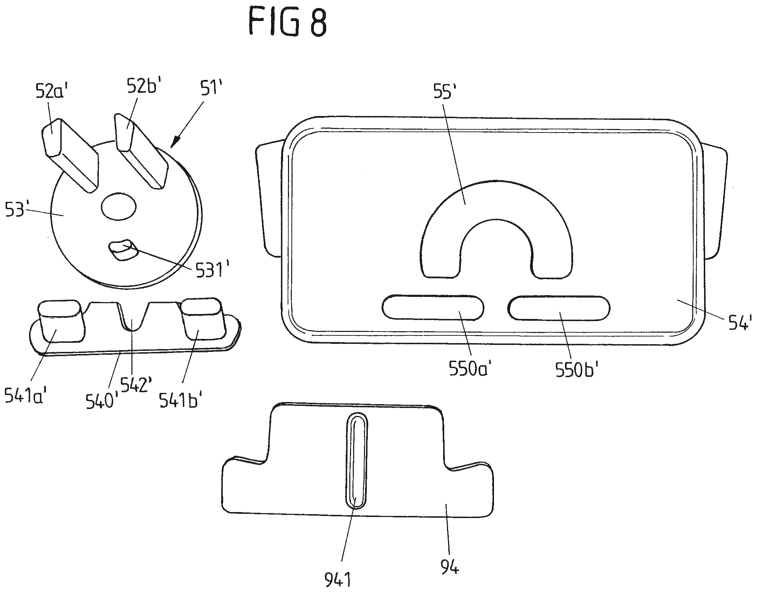

The components of the actuating device 5' shown in Fig. 7A-C are shown in Fig. 8. As can be seen, the rotatable member 51' comprises a circular base body 53' which the arms 52a', 52b' protrude from. The additional movable arms 541a', 541b' protrude from a separate longitudinal base body 540'. The base body 540' in an upper edge comprises a V- or U- shaped recess 542' into which an eccentric pin 531' extending from the circular base body 53' engages. A rotation of the base body 53' (i.e. of the arms 52a', 52b') thus generates the (opposite) linear movement of the longitudinal base body 540' and thus of the additional movable arms 541a', 541b'.

As further shown in Fig. 8, the adapter plate 94 comprises a notch 941 which receives a backside pin (not visible in Fig. 8) of the rotatable member 51' such that a linear movement of the adapter plate 94 is converted into a rotational movement of the rotatable member 51' (and a linear movement of the additional movable arms 541a', 541b') as set forth above.

### Reference signs

- 1: blood preparation device
- 2: opening device
- 3: universal mount
- 4, 4', 5, 5', 61, 71, 81, 9: actuating device
- 11: housing
- 12: attachment region
- 21: drive mechanism
- 22: rotating plate
- 23a, 23b: stud
- 31: bolt
- 32: bearing surface
- 41a, 41b, 41a', 41b': arm
- 42, 42': guiding element
- 51, 51', 82, 83, 91: rotatable member
- 52a, 52b, 52a', 52b', 612a,: arm
- 612b, 712a, 712b, 92a, 92b 53, 53', 93, 611, 711: base body
- 54, 54', 95: housing
- 55, 55', 955: opening
- 94: adapter plate
- 101: closure member
- 111: opening
- 211: motor
- 212: shaft
- 421: opening
- 511: eccentric backside pin
- 531': eccentric pin
- 540': base body
- 541a, 541b, 613a, 613b,: fixed protrusion
- 713a, 713b 541a', 541b': movable arm
- 542': recess
- 550a', 550b': recess housing
- 821, 822: base body
- 822a, 822b: arm first base body
- 832a, 832b: arm second base body
- 941: longitudinal recess

## Claims

1. Opening device assembly comprising an opening device for opening a closure member, in particular a closure member of a medical container, and an actuating device (4, 4', 5, 5', 61, 71, 81, 9) for interacting with the closure member (101), the opening device comprising:
- a drive mechanism (21) for driving the actuating device (4, 4', 5, 5', 61, 71, 81, 9), wherein using the drive mechanism (21) the actuating device can be moved from an initial position into an engagement position in order to open the closure member (101), and
- a connecting device (3) for detachably connecting the actuating device (4, 4', 5, 5', 61, 71, 81, 9) to the drive mechanism (21) such that the actuating device (4, 4', 5, 5', 61, 71, 81, 9) can be interchanged with another actuating device,
**characterized in that**
the connecting device (3) is configured in such a way that at least a first and a second type of an actuating device (4, 4', 5, 5', 61, 71, 81, 9) can be operably connected to the drive mechanism (21), the first type using a rotational motion and the second type using a linear motion for interacting with the closure member (101), wherein
the actuating device comprises at least one actuating element (41a, 41b, 41a', 41b', 52a, 52b, 52a', 52b', 612a, 612b, 712a, 712b, 92a, 92b) configured for interacting with the closure member (101) and an adapter element (94) in the form of an adapter plate that is set into a linear motion by the drive mechanism (21), wherein
the drive mechanism (21) comprises at least one stud (23a, 23b) arranged eccentrically relative to a shaft (212) of the drive mechanism (21), the stud (23a, 23b) interacting with the adapter element (94) when the actuating device (4, 4', 5, 5', 61, 71, 81, 9) is connected to the drive mechanism (21), and wherein the actuating element (41a, 41b, 41a', 41b', 52a, 52b, 52a', 52b', 612a, 612b, 712a, 712b, 92a, 92b) is coupled to the adapter element (94) in such a way that the linear motion of the adapter element (94) is transferred to the actuating element or causes a rotational motion of the actuating element, and wherein
the actuating device comprises a rotatable member (51, 91) having two actuating elements in the form of arms and an eccentric pin (511, 911) that engages a longitudinal recess (941) of the adapter element (94) such that a to and fro movement of the adapter element (94) causes a back and forth rotation of the rotatable member (51, 91) and thus of the arms.

2. Opening device assembly as claimed in claim 1, , wherein the connecting device (3) comprises at least one longitudinal element (31) fixedly connected to the drive mechanism (21) and a corresponding opening of the actuating device (4, 4', 5, 5', 61, 71, 81, 9) into which the longitudinal element (31) is inserted when connecting the actuating device (4, 4', 5, 5', 61, 71, 81, 9) to the drive mechanism (21).

3. Opening device assembly as claimed in claim 1 or 2, wherein the drive mechanism (21) comprises two studs (23a, 23b) arranged eccentrically relative to a shaft (212) of the drive mechanism (21), wherein one of the studs (23a, 23b) is shorter than the other one.

4. Opening device assembly according to any of the preceding claims, wherein the adapter element (94) is an adapter plate and comprises a longitudinal recess (941) into which an eccentric pin (911) of a rotatable member (91) of the actuating device (4, 4', 5, 5', 61, 71, 81, 9) engages such that the rotatable member (91) can be arranged in different positions relative to the adapter element (94) while the pin (911) still engages the longitudinal recess (941).

5. Opening device assembly as claimed in any of the preceding claims, wherein the actuating device is a first actuating device and the opening device assembly comprises a second actuating device, wherein
- the first and the second actuating device (4, 4', 5, 5', 61, 71, 81, 9) can be interchangeably connected to the drive mechanism (21) of the opening device via the connecting device (3),
- wherein the second actuating device comprises at least one actuating element (41a, 41b, 41a', 41b') configured for interacting with the closure member (101) and at least one connecting element for being detachably connected to the drive mechanism (21) of the opening device (2), and wherein the second actuating device (4, 4') is configured in such a way that a rotational movement provided by the drive mechanism (21) is converted into a linear movement of the actuating element (41a, 41b, 41a', 41b').

6. Medical device, in particular a blood preparation device, comprising an opening device assembly as claimed in any of the preceding claims.

7. Actuating device for use with a drive mechanism (21) of an opening device (2) of an opening device assembly according to one of the claims 1 to 5 for opening a closure member (101), comprising
- at least one actuating element (52a, 52b, 52a', 52b', 612a, 612b, 712a, 712b, 92a, 92b) configured for interacting with the closure member (101) and at least one connecting element for being detachably connected to the drive mechanism (21) of the opening device (2), wherein the actuating device (5, 5', 61, 71, 81, 9) is configured in such a way that a rotational movement provided by the drive mechanism (21) causes a rotational movement of the actuating element (52a, 52b, 52a', 52b', 612a, 612b, 712a, 712b, 92a, 92b), and
- - an adapter element (94) in the form of an adapter plate that is set into a linear motion by the drive mechanism (21),
**characterized by**
a rotatable member (51, 91) having two actuating elements in the form of arms and an eccentric pin (511, 911) that engages a longitudinal recess (941) of the adapter element (94) such that a to and fro movement of the adapter element (94) causes a back and forth rotation of the rotatable member (51, 91) and thus of the arms.

8. Actuating device according to claim 7, further comprising a first pair of actuating elements (52a', 52b') that is set in a rotational motion by a rotational movement provided by the drive mechanism (21) and a second pair of actuating elements (541a', 541b') that is set in a linear motion by a rotational movement provided by the drive mechanism (21).

9. Method for opening a closure member, in particular a closure member of a medical container, comprising the steps of:
- providing an opening device assembly as claimed in claim 5;
- choosing one of the actuating devices (4, 4', 5, 5', 61, 71, 81, 9) of the opening device assembly dependent on the configuration of the closure member (101);
- connecting the chosen actuating device (4, 4', 5, 5', 61, 71, 81, 9) to the drive mechanism (21); and
- opening the closure member (101) using the opening device (2) of the opening device assembly.

## Patentansprüche

1. Öffnungsvorrichtungsanordnung, aufweisend eine Öffnungsvorrichtung zum Öffnen eines Verschlusselements, insbesondere eines Verschlusselements eines medizinischen Behälters, und eine Betätigungsvorrichtung (4, 4', 5, 5', 61, 71, 81, 9) zur Interaktion mit dem Verschlusselement (101), wobei die Öffnungsvorrichtung das Folgende aufweist:
- einen Antriebsmechanismus (21) zum Antreiben der Betätigungsvorrichtung (4, 4', 5, 5', 61, 71, 81, 9), wobei durch Verwendung des Antriebsmechanismus (21) die Betätigungsvorrichtung aus einer Anfangsstellung in eine Eingriffsstellung zum Öffnen des Verschlusselements (101) bewegt werden kann, und
- eine Verbindungsvorrichtung (3) zum lösbaren Verbinden der Betätigungsvorrichtung (4, 4', 5, 5', 61, 71, 81, 9) mit dem Antriebsmechanismus (21), so dass die Betätigungsvorrichtung (4, 4', 5, 5', 61, 71, 81, 9) gegen eine andere Betätigungsvorrichtung ausgetauscht werden kann,
**dadurch gekennzeichnet, dass**
die Verbindungsvorrichtung (3) derart ausgelegt ist, dass mindestens eine erste und eine zweite Art von Betätigungsvorrichtung (4, 4', 5, 5', 61, 71, 81, 9) mit dem Antriebsmechanismus (21) betriebsfähig verbunden werden kann, wobei die erste Art eine Drehbewegung verwendet und die zweite Art eine lineare Bewegung für die Interaktion mit dem Verschlusselement (101) verwendet, wobei
die Betätigungsvorrichtung mindestens ein Betätigungselement (41a, 41b, 41a', 41b', 52a, 52b, 52a', 52b', 612a, 612b, 712a, 712b, 92a, 92b) aufweist, das für eine Interaktion mit dem Verschlusselement (101) ausgelegt ist, und ein Adapterelement (94) in Form einer Adapterplatte, die vom Antriebsmechanismus (21) in eine lineare Bewegung versetzt wird, wobei
der Antriebsmechanismus (21) mindestens einen Stift (23a, 23b) aufweist, der bezüglich einer Welle (212) des Antriebsmechanismus (21) exzentrisch angeordnet ist, wobei der Stift (23a, 23b) mit dem Adapterelement (94) interagiert, wenn die Betätigungsvorrichtung (4, 4', 5, 5', 61, 71, 81, 9) mit dem Antriebsmechanismus (21) verbunden ist, und wobei das Betätigungselement (41a, 41b, 41a', 41b', 52a, 52b, 52a', 52b', 612a, 612b, 712a, 712b, 92a, 92b) auf eine solche Weise mit dem Adapterelement (94) gekoppelt ist, dass die lineare Bewegung des Adapterelements (94) auf das Betätigungselement übertragen wird oder eine Drehbewegung des Betätigungselements verursacht, und wobei
die Betätigungsvorrichtung ein drehbares Element (51, 91) mit zwei Betätigungselementen in Form von Armen und einem exzentrischen Bolzen (511, 911) aufweist, der eine Längsaussparung (941) des Adapterelements (94) in Eingriff nimmt, so dass eine hin- und hergehende Bewegung des Adapterelements (94) eine Vorwärts- und Rückwärtsdrehung des drehbaren Elements (51, 91) und somit der Arme verursacht.

2. Öffnungsvorrichtungsanordnung nach Anspruch 1, wobei die Verbindungsvorrichtung (3) mindestens ein Längselement (31), das fest mit dem Antriebsmechanismus (21) verbunden ist, und eine entsprechende Öffnung der Betätigungsvorrichtung (4, 4', 5, 5', 61, 71, 81, 9) aufweist, in die das Längselement (31) bei der Verbindung der Betätigungsvorrichtung (4, 4', 5, 5', 61, 71, 81, 9) mit dem Antriebsmechanismus (21) eingeführt wird.

3. Öffnungsvorrichtungsanordnung nach Anspruch 1 oder 2, wobei der Antriebsmechanismus (21) zwei Stifte (23a, 23b) aufweist, die bezüglich einer Welle (212) des Antriebsmechanismus (21) exzentrisch angeordnet sind, wobei einer der Stifte (23a, 23b) kürzer als der andere ist.

4. Öffnungsvorrichtungsanordnung nach einem der vorhergehenden Ansprüche, wobei das Adapterelement (94) eine Adapterplatte ist und eine Längsaussparung (941) aufweist, in die ein exzentrischer Bolzen (911) eines drehbaren Elements (91) der Betätigungsvorrichtung (4, 4', 5, 5', 61, 71, 81, 9) eingreift, so dass das drehbare Element (91) in verschiedenen Stellungen bezüglich des Adapterelements (94) angeordnet werden kann, während der Bolzen (911) noch mit der Längsaussparung (941) in Eingriff steht.

5. Öffnungsvorrichtungsanordnung nach einem der vorhergehenden Ansprüche, wobei die Betätigungsvorrichtung eine erste Betätigungsvorrichtung ist und die Öffnungsvorrichtungsanordnung eine zweite Betätigungsvorrichtung aufweist, wobei
- die erste und die zweite Betätigungsvorrichtung (4, 4', 5, 5', 61, 71, 81, 9) austauschbar mit dem Antriebsmechanismus (21) der Öffnungsvorrichtung über die Verbindungsvorrichtung (3) verbunden werden können,
- wobei die zweite Betätigungsvorrichtung mindestens ein Betätigungselement (41a, 41b, 41a', 41b'), das für eine Interaktion mit dem Verschlusselement (101) ausgelegt ist, und mindestens ein Verbindungselement zur lösbaren Verbindung mit dem Antriebsmechanismus (21) der Öffnungsvorrichtung (2) aufweist, und wobei die zweite Betätigungsvorrichtung (4, 4') auf eine solche Weise ausgelegt ist, dass eine vom Antriebsmechanismus (21) bereitgestellte Drehbewegung in eine lineare Bewegung des Betätigungselements (41a, 41b, 41a', 41b') umgewandelt wird.

6. Medizinische Vorrichtung, insbesondere eine Blutaufbereitungsvorrichtung, aufweisend eine Öffnungsvorrichtungsanordnung nach einem der vorhergehenden Ansprüche.

7. Betätigungsvorrichtung zur Verwendung mit einem Antriebsmechanismus (21) einer Öffnungsvorrichtung (2) einer Öffnungsvorrichtungsanordnung nach einem der Ansprüche 1 bis 5 zum Öffnen eines Verschlusselements (101), aufweisend
- mindestens ein Betätigungselement (52a, 52b, 52a', 52b', 612a, 612b, 712a, 712b, 92a, 92b), das für eine Interaktion mit dem Verschlusselement (101) ausgelegt ist, und mindestens ein Verbindungselement zur lösbaren Verbindung mit dem Antriebsmechanismus (21) der Öffnungsvorrichtung (2), wobei die Betätigungsvorrichtung (5, 5', 61, 71, 81, 9) auf eine solche Weise ausgelegt ist, dass eine vom Antriebsmechanismus (21) bereitgestellte Drehbewegung eine Drehbewegung des Betätigungselements (52a, 52b, 52a', 52b', 612a, 612b, 712a, 712b, 92a, 92b) verursacht, und
- ein Adapterelement (94) in Form einer Adapterplatte, die vom Antriebsmechanismus (21) in eine lineare Bewegung versetzt wird,
**gekennzeichnet durch**
ein drehbares Element (51, 91) mit zwei Betätigungselementen in Form von Armen und einem exzentrischen Bolzen (511, 911), der mit einer Längsaussparung (941) des Adapterelements (94) in Eingriff steht, so dass eine hin- und hergehende Bewegung des Adapterelements (94) eine Vorwärts- und Rückwärtsdrehung des drehbaren Elements (51, 91) und somit der Arme verursacht.

8. Betätigungsvorrichtung nach Anspruch 7, ferner aufweisend ein erstes Paar von Betätigungselementen (52a', 52b'), die durch eine vom Antriebsmechanismus (21) bereitgestellte Drehbewegung in eine Drehbewegung versetzt werden, und ein zweites Paar von Betätigungselementen (541a', 541b'), die durch eine vom Antriebsmechanismus (21) bereitgestellte Drehbewegung in eine lineare Bewegung versetzt werden.

9. Verfahren zum Öffnen eines Verschlusselements, insbesondere eines Verschlusselements eines medizinischen Behälters, aufweisend die folgenden Schritte:
- Bereitstellen einer Öffnungsvorrichtungsanordnung nach Anspruch 5;
- Auswählen einer der Betätigungsvorrichtungen (4, 4', 5, 5', 61, 71, 81, 9) der Öffnungsvorrichtungsanordnung in Abhängigkeit von der Konfiguration des Verschlusselements (101);
- Verbinden der ausgewählten Betätigungsvorrichtung (4, 4', 5, 5', 61, 71, 81, 9) mit dem Antriebsmechanismus (21); und
- Öffnen des Verschlusselements (101) unter Verwendung der Öffnungsvorrichtung (2) der Öffnungsvorrichtungsanordnung.

## Revendications

1. Ensemble de dispositif d'ouverture comprenant un dispositif d'ouverture pour ouvrir un organe de fermeture, en particulier un organe de fermeture d'un récipient médical, et un dispositif d'actionnement (4, 4', 5, 5', 61, 71, 81, 9) destiné à interagir avec l'organe de fermeture (101), le dispositif d'ouverture comprenant:
- un mécanisme d'entraînement (21) pour entraîner le dispositif d'actionnement (4, 4', 5, 5', 61, 71, 81, 9), en utilisant du mécanisme d'entraînement (21), le dispositif d'actionnement pouvant être déplacé d'une position initiale dans une position d'engagement afin d'ouvrir l'organe de fermeture (101), et
- un dispositif de connexion (3) pour relier de manière détachable le dispositif d'actionnement (4, 4', 5, 5', 61, 71, 81, 9) au mécanisme d'entraînement (21) de telle sorte que le dispositif d'actionnement (4, 4', 5, 5', 61, 71, 81, 9) puisse être échangé avec un autre dispositif d'actionnement,
**caractérisé en ce que**
le dispositif de connexion (3) est configuré de telle sorte qu'au moins un premier et un deuxième type de dispositif d'actionnement (4, 4', 5, 5', 61, 71, 81, 9) puissent être connectés fonctionnellement au mécanisme d'entraînement (21), le premier type utilisant un mouvement de rotation et le deuxième type utilisant un mouvement linéaire pour interagir avec l'organe de fermeture (101),
dans lequel le dispositif d'actionnement comprenant au moins un élément d'actionnement (41a, 41b, 41a', 41b', 52a, 52b, 52a', 52b', 612a, 612b, 712a, 712b, 92a, 92b) configuré pour interagir avec l'organe de fermeture (101) et un élément adaptateur (94) sous la forme d'une plaque d'adaptateur qui est mise en mouvement linéaire par le mécanisme d'entraînement (21), dans lequel
le mécanisme d'entraînement (21) comprenant au moins un goujon (23a, 23b) disposé de manière excentrique par rapport à un arbre (212) du mécanisme d'entraînement (21), le goujon (23a, 23b) interagissant avec l'élément adaptateur (94) lorsque le dispositif d'actionnement (4, 4', 5, 5', 61, 71, 81, 9) est connecté au mécanisme d'entraînement (21), et l'élément d'actionnement (41a, 41b, 41a', 41b', 52a, 52b, 52a', 52b', 612a, 612b, 712a, 712b, 92a, 92b) étant accouplé à l'élément adaptateur (94) de telle sorte que le mouvement linéaire de l'élément adaptateur (94) soit transféré à l'élément d'actionnement ou provoque un mouvement de rotation de l'élément d'actionnement, et
le dispositif d'actionnement comprenant un organe rotatif (51, 91) ayant deux éléments d'actionnement sous la forme de bras et une goupille excentrique (511, 911) qui s'engage dans un renfoncement longitudinal (941) de l'élément adaptateur (94) de telle sorte qu'un mouvement de va-et-vient de l'élément adaptateur (94) provoque une rotation d'avant en arrière de l'organe rotatif (51, 91) et par conséquent des bras.

2. Ensemble de dispositif d'ouverture selon la revendication 1, dans lequel le dispositif de connexion (3) comprend au moins un élément longitudinal (31) connecté fixement au mécanisme d'entraînement (21) et une ouverture correspondante du dispositif d'actionnement (4, 4', 5, 5', 61, 71, 81, 9) dans laquelle l'élément longitudinal (31) est inséré lors de la connexion du dispositif d'actionnement (4, 4', 5, 5', 61, 71, 81, 9) au mécanisme d'entraînement (21).

3. Ensemble de dispositif d'ouverture selon la revendication 1 ou 2, dans lequel le mécanisme d'entraînement (21) comprend deux goujons (23a, 23b) disposés de manière excentrique par rapport à un arbre (212) du mécanisme d'entraînement (21), l'un des goujons (23a, 23b) étant plus court que l'autre.

4. Ensemble de dispositif d'ouverture selon l'une quelconque des revendications précédentes, dans lequel l'élément adaptateur (94) est une plaque d'adaptateur et comprend un renfoncement longitudinal (941) dans lequel s'engage une goupille excentrique (911) d'un organe rotatif (91) du dispositif d'actionnement (4, 4', 5, 5', 61, 71, 81, 9) de telle sorte que l'organe rotatif (91) puisse être disposé dans différentes positions par rapport à l'élément adaptateur (94) tandis que la goupille (911) s'engage toujours dans le renfoncement longitudinal (941).

5. Ensemble de dispositif d'ouverture selon l'une quelconque des revendications précédentes, dans lequel le dispositif d'actionnement est un premier dispositif d'actionnement et l'ensemble de dispositif d'ouverture comprend un deuxième dispositif d'actionnement,
- le premier et le deuxième dispositif d'actionnement (4, 4', 5, 5', 61, 71, 81, 9) pouvant être connectés de manière interchangeable au mécanisme d'entraînement (21) du dispositif d'ouverture par le biais du dispositif de connexion (3),
- le deuxième dispositif d'actionnement comprenant au moins un élément d'actionnement (41a, 41b, 41a', 41b') configuré pour interagir avec l'organe de fermeture (101) et au moins un élément de connexion prévu pour être connecté de manière détachable au mécanisme d'entraînement (21) du dispositif d'ouverture (2), et dans lequel le deuxième dispositif d'actionnement (4, 4') étant configuré de telle sorte qu'un mouvement de rotation fourni par le mécanisme d'entraînement (21) soit converti en un mouvement linéaire de l'élément d'actionnement (41a, 41b, 41a', 41b').

6. Dispositif médical, en particulier dispositif de préparation de sang, comprenant un ensemble de dispositif d'ouverture selon l'une quelconque des revendications précédentes.

7. Dispositif d'actionnement pour l'utilisation avec un mécanisme d'entraînement (21) d'un dispositif d'ouverture (2) d'un ensemble de dispositif d'ouverture selon l'une des revendications 1 à 5, pour ouvrir un organe de fermeture (101), comprenant:
- au moins un élément d'actionnement (52a, 52b, 52a', 52b', 612a, 612b, 712a, 712b, 92a, 92b) configuré pour interagir avec l'organe de fermeture (101) et au moins un élément de connexion prévu pour être connecté de manière détachable au mécanisme d'entraînement (21) du dispositif d'ouverture (2), dans lequel le dispositif d'actionnement (5, 5', 61, 71, 81, 9) étant configuré de telle sorte qu'un mouvement de rotation fourni par le mécanisme d'entraînement (21) provoque un mouvement de rotation de l'élément d'actionnement (52a, 52b, 52a', 52b', 612a, 612b, 712a, 712b, 92a, 92b) et
- un élément adaptateur (94) sous la forme d'une plaque d'adaptateur qui est mise en mouvement linéaire par le mécanisme d'entraînement (21),
**caractérisé par**
un organe rotatif (51, 91) ayant deux éléments d'actionnement sous la forme de bras et une goupille excentrique (511, 911) qui s'engage dans un renfoncement longitudinal (941) de l'élément adaptateur (94) de telle sorte qu'un mouvement de va-et-vient de l'élément adaptateur (94) provoque une rotation d'avant en arrière de l'organe rotatif (51, 91) et par conséquent des bras.

8. Dispositif d'actionnement selon la revendication 7, comprenant en outre une première paire d'éléments d'actionnement (52a', 52b') qui est mise en mouvement de rotation par un mouvement de rotation fourni par le mécanisme d'entraînement (21) et une deuxième paire d'éléments d'actionnement (541a', 541b') qui est mise en mouvement linéaire par un mouvement de rotation fourni par le mécanisme d'entraînement (21).

9. Procédé pour ouvrir un organe de fermeture, en particulier un organe de fermeture d'un récipient médical, comprenant les étapes suivantes:
- fourniture d'un ensemble de dispositif d'ouverture selon la revendication 5;
- sélection d'un des dispositifs d'actionnement (4, 4', 5, 5', 61, 71, 81, 9) de l'ensemble de dispositif d'ouverture en fonction de la configuration de l'organe de fermeture (101);
- connexion du dispositif d'actionnement sélectionné (4, 4', 5, 5', 61, 71, 81, 9) au mécanisme d'entraînement (21); et
- ouverture de l'organe de fermeture (101) en utilisant le dispositif d'ouverture (2) de l'ensemble de dispositif d'ouverture.
